(19) Europäisches Patentamt
European Patent Office
Office européen des brevets

(11) Veröffentlichungsnummer: **0 264 660 B1**

(12) **EUROPÄISCHE PATENTSCHRIFT**

(45) Veröffentlichungstag der Patentschrift: **17.06.92**

(51) Int. Cl.$^5$: **A61K 6/00**

(21) Anmeldenummer: **87114086.9**

(22) Anmeldetag: **26.09.87**

# (54) Verwendung eines Dentalwerkstoffs zur Bekämpfung von Karies und Parodontose.

(30) Priorität: **11.10.86 DE 3634697**

(43) Veröffentlichungstag der Anmeldung:
**27.04.88 Patentblatt 88/17**

(45) Bekanntmachung des Hinweises auf die Patenterteilung:
**17.06.92 Patentblatt 92/25**

(84) Benannte Vertragsstaaten:
**AT CH DE FR GB IT LI SE**

(56) Entgegenhaltungen:
**EP-A- 0 128 655**
**EP-A- 0 223 245**
**US-A- 3 390 456**

**Römpp's Chemie Lexikon, 8. Aufl.; S. 612, 4263;**

**J. Dent. Res., Band 64, 1985; S. 1356-1360;**

(73) Patentinhaber: **Ivoclar AG**
**FL-9494 Schaan(LI)**

(72) Erfinder: **Michl, Rudolf J.**
**Im Fetzer 49**
**LI-9494 Schaan(LI)**

(74) Vertreter: **UEXKÜLL & STOLBERG Patentanwälte**
**Beselerstrasse 4**
**W-2000 Hamburg 52(DE)**

**EP 0 264 660 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Gegenstände der Ansprüche 1 bis 8.

Trotz jahrzehntelanger Untersuchungen und zahlreicher Versuche zur prophylaktischen und therapeutischen Behandlung von Karies und Parodontose stellen diese ein noch immer unbewältigtes Problem dar.

Die medikamentöse Behandlung wurde bisher vorwiegend mit Fluoriden vorgenommen; vgl. Dr. Hans Joachim Schmidt, "Zahnhalskariesprophylaxe durch Fluoride", Dr. Alfred Hüthig Verlag, Heidelberg, 2. Aufl. (1967). Die Verabreichung von Fluortabletten ist jedoch nur bis zum Abschluß der Zahnbildung, d.h. bis etwa zum 12. Lebensjahr sinnvoll. Nach diesem Zeitpunkt kommt in erster Linie die lokale Applikation von Wirkstoffen zur prophylaktischen und therapeutischen Behandlung von Karies und Parodontose in Betracht.

Fluoride sowie sonstige Wirkstoffe und insbesondere antimikrobiell wirksame Substanzen werden bisher überwiegend in Mund- und Zahnpflegemittel eingearbeitet und bei der täglichen Mundhygiene zur Einwirkung gebracht. Die dabei erreichte Einwirkungsdauer ist jedoch kurz, da die Wirkstoffe durch den permanenten Speichelfluß und die Nahrungsaufnahme sehr schnell verdünnt und ausgespült werden.

Zur Vermeidung dieser Nachteile ist bereits vorgeschlagen worden, zur therapeutischen Behandlung von Parodontose antimikrobiell wirksame Substanzen wie Chlorhexidin in eine Folie eingebettet als System mit verzögerter Wirkstoffabgabe in das Parodontium einzubringen; vgl. Coventry, J. und Newman, H.N. J. Clin. Periodontol. 9, 129-133 (1982) und Friedman, M. und Golomb, G. J. Periodontol. 17, 323-328 (1982). Ein weiteres, Chlorhexidin enthaltendes System mit verzögerter Wirkstoffabgabe zur Anwendung im Zahnbereich wird von T.E. Balanyk und H.J. Sandham in J. Dent. Res. 64, 1356-1360 (1985) beschrieben. Es handelt sich um eine Lösung eines Chlorhexidinsalzes in Benzoin-Harz, die beim Auftrocknen einen festen Film bildet, aus dem der Wirkstoff verzögert abgegeben wird. Die beschriebenen in vitro Versuche zeigen allerdings, daß nach 14 Tagen nur 3 von 10 mg Chlorhexidin aus dem System abgegeben waren und der restliche Wirkstoff nicht verfügbar war. Ferner ergibt sich aus der Tabelle 1, daß die Antiseptika Thymol und Jod am wenigsten wirkungsvoll sind.

Es ist Aufgabe der Erfindung, ein Mittel zur wirksamen prophylaktischen und therapeutischen Bekämpfung von Karies und Parodontose und insbesondere von Zahnhalskaries zu schaffen, welches über längere Zeit oder permanent in der Mundhöhle verbleibt, somit eine längere Einwirkungsdauer als Mund- und Zahnpflegemittel besitzt und bei Vermeidung der oben beschriebenen Nachteile eine verbesserte antimikrobielle Wirksamkeit aufweist sowie eine Härtung des befallenen Dentins herbeiführt.

Die Aufgabe wird erfindungsgemäß gelöst.

Überraschenderweise hat es sich gezeigt, daß durch die erfindungsgemäße Verwendung einer Kombination aus den antimikrobiellen Wirkstoffen Thymol und/oder Carvacrol der Formeln

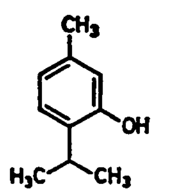 , 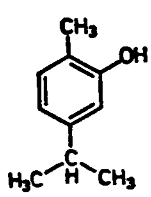

und dem antimikrobiellen Wirkstoff Chlorhexidin der Formel

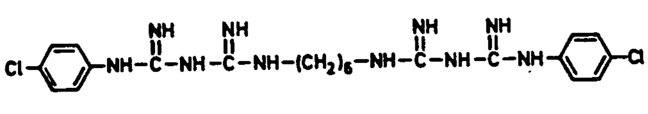

und/oder aus physiologisch verträglichen Salzen derselben in einem Dentalwerkstoff, wobei das Thymol und/oder Carvacrol oder deren Salze in einer Menge von 10 bis 0,05 Gew.% und das Chlorhexidin oder dessen Salze in einer Menge von 2 bis 0,01 Gew.%, jeweils bezogen auf den Dentalwerkstoff vorliegen, eine Steigerung der mikrobiciden Wirksamkeit dieser Substanzen erzielt wird. Zusätzlich wird in überraschender Weise durch Verwendung der Wirkstoffkombination aus Thymol und/oder Carvacrol und Chlorhexidin oder deren Salzen in einem Dentalwerkstoff eine signifikante und selektive Härtung von kariösem Dentin erreicht.

Die Wirkstoffkombination kann in jeglichem dentalen Werkstoff als Träger verwendet werden, der

härtbar ist, bzw. hart wird und aus dem die Wirkstoffe herausdiffundieren können. Vorzugsweise wird ein Werkstoff verwendet, aus dem die Wirkstoffe in das Dentin diffundieren können.

Erfindungsgemäß soll der Begriff "Dentalwerkstoff" solche Trägermaterialien umfassen, die über einen gewissen Zeitraum von Stunden bis Jahren in der Mundhöhle verbleiben.

Gemäß einer bevorzugten Ausführungsform der Erfindung wird als Träger eine Lösung von Kolophonium in einem organischen Lösungsmittel verwendet, wobei sich Ethanol, Propanol sowie Amylacetat als Lösungsmittel besonders bewährt haben. Gute Ergebnisse wurden auch mit beispielsweise 1,2-Dichlorethylen erhalten. Die Lösungen können durch Pinseln, Tropfen oder Sprayen auf den Zahnhals aufgebracht werden und bilden dort einen fest haftenden Lack, der als Wirkstoff-Reservoir dient und aus dem die Wirkstoffkombination gleichmäßig und vollständig in das Dentin diffundiert.

Als wirkungsvoll hat es sich ebenfalls erwiesen, den Dentalwirkstoff, beispielsweise als Zahnlack auf ein festes Substrat aufzubringen, wo er gegebenenfalls aushärten kann. Erfindungsgemäß umfaßt der Begriff festes Substrat beispielsweise künstliche Zähne, Kronen und Brücken, Zahnstocher, Zahnseide und ähnliche Geräte und Vorrichtungen, die mit dem erfindungsgemäßen Dentalwerkstoff präpariert und in die Mundhöhle eingebracht werden können und nach deren Einsatz bzw. Gebrauch die erfindungsgemäße Kombination innerhalb der Mundhöhle ihre Wirkung entfalten kann. So kann beispielsweise die Spitze eines Zahnstochers mit Zahnlack präpariert und die erfindungsgemäße Kombination gleichzeitig mit dem Reinigungsvorgang aufgetragen werden.

Gemäß weiterer Ausführungsformen der Erfindung wird die Wirkstoffkombination in Dentalwerkstoffe wie Zement, Unterfüllungs-, Füllungs-, Versiegelungs-, Befestigungs-, Prothesen- oder Kronen- und Brükkenmaterialien sowie Materialien zur Herstellung von künstlichen Zähnen oder in Haftmitteln (Adhesive) eingearbeitet.

Das Thymol und/oder Carvacrol wird, vorzugsweise in einer Menge von 5 bis 0,25 Gew.% und in besonders bevorzugter Weise von 2 bis 0,5 Gew.% jeweils bezogen auf den Dentalwerkstoff verwendet. Das Chlorhexidin, vorzugsweise in einer Menge von 1 bis 0,05 Gew.% und in besonders bevorzugter Weise 0,5 bis 0,1 Gew.% ebenfalls bezogen auf den Dentalwerkstoff verwendet.

Die genannten Wirkstoffe können sowohl als solche sowie als jegliches ihrer physikalisch verträglichen Salze verwendet werden. Als Salze des Chlorhexidins kommen beispielsweise Chlorhexidin-di-D-gluconat, Chlorhexidin-dihydrochlorid sowie Chlorhexidin-diacetat in Betracht. Erfindungsgemäß können ferner verwandte Verbindungen des Chlorhexidins wie Alexidin, Picloxydin oder Vantocil sowie des Thymols wie beispielsweise Aristol verwendet werden.

Der erfindungsgemäß verwendete Dentalwerkstoff kann ferner Fluor-Ionen beispielsweise als Natriumfluorid in Mengen von beispielsweise 5 bis 0,05 und vorzugsweise 2 bis 0,1 Gew.% bezogen auf die Gesamtzubereitung enthalten. Anstelle von Fluor-Ionen können auch Zink-Ionen, beispielsweise als Chlorid oder als Acetat, Zinn-Ionen, beispielsweise als Chlorid, zweiwertige Kupfer-Ionen, beispielsweise als Kupfernitrat und Molybdän-Ionen, beispielsweise als $Na_2MoO_4.2H_2O$ oder Mischungen vorhanden sein.

Der erfindungsgemäß verwendete Dentalwerkstoff wirkt sowohl prophylaktisch als auch therapeutisch gegen Bakterien, wobei die Wirkungsdauer wesentlich von dem gewählten Trägersystem abhängt. Wird beispielsweise gemäß einer besonders bevorzugten Ausführungsform der Erfindung eine Lösung von Kolophonium in hochprozentigem Ethanol verwendet, so kann diese als Lack insbesondere auf den Zahnhals gepinselt, getropft oder gesprüht werden. Dieser Lack haftet eine gewisse Zeit lang und gibt während dieser Zeit die enthaltenen Wirkstoffe nahezu vollständig an den Zahnhals ab. Obwohl der Lack nach verhältnismäßig kurzer Zeit durch den Speichel abgelöst wird, hält seine therapeutische Wirkung über längere Zeit an. Überraschenderweise findet offenbar eine schnelle Diffusion der erfindungesgemäßen Wirkstoffkombination in das Dentin statt und es erfolgt von dort aus eine gleichmäßige Wirkstoffabgabe. Dies war in keiner Weise vorhersehbar. Die Anfälligkeit des Zahnhalses gegenüber Bakterien wird damit erheblich verringert und - soweit bereits ein Befall stattgefunden hat - wird das Dentin gehärtet und gegenüber einer Ausbreitung des Befalls stabilisiert.

Als Trägermaterialien für Zahnlacke kommen ferner Schellack, Benzoinharz, Polyvinylpyrrolidon oder andere künstliche oder natürliche Harze in Betracht, die in einem Lösungsmittel lösbar sind und die nach Verdunsten des Lösungsmittels hart werden.

Wird die erfindungsgemäße Wirkstoffkombination in Zemente oder sonstige Dentalmaterialien eingearbeitet, so kann eine gleichmäßige Wirkstoffabgabe über mehrere Wochen und Monate erreicht werden. Beispielsweise kann die erfindungsgemäße Wirkstoffkombination in ein Unterfüllungsmaterial eingearbeitet werden, wobei die ständige Wirkstoffabgabe wirkungsvoll das Entstehen von Sekundärkaries verhindert.

Ebenso ist es denkbar, die antibakterielle Wirkstoffkombination in ein lichthärtendes, bzw. konventionell aushärtendes Füllungsmaterial einzuarbeiten, um die Entstehung von Sekundärkaries zu verlangsamen.

Ferner können die erfindungsgemäß verwendeten Wirkstoffe in Prothesenmaterialien eingearbeitet

werden, wobei die fertige Prothese konstant kleinste Mengen der Wirkstoffe abgibt und somit der Ausbreitung von Plaque, die dann zu Karies an den noch vorhandenen Zähnen führt, entgegenwirkt.

Eine ähnliche Wirkung hat der Einsatz der Erfindung in künstlichen Zähnen.

Unterfüllungsmaterialien, Füllungsmaterialien, Prothesenmaterialien, künstliche Zähne etc. sind dem Fachmann geläufig und nicht Gegenstand der Erfindung. Solche Dentalwerkstoffe sind beispielsweise in der DE-OS 24 03 211, 27 49 564 und US-PS 3 047 408 offenbart.

Die Erfindung wird nachfolgend anhand von Beispielen erläutert.

## Beispiel 1

Herstellung eines Lacks

3 g Kolophonium wurden in einen Kolben gegeben, mit 6 ml reinem (96) %-igem Ethanol versetzt, und nach Verschließen des Kolbens 24 Stunden lang zum Lösen geschüttelt.

Zu der Lösung wurden 2 ml destilliertes Wasser gegeben und anschließend 0,1 g Thymol, 0,02 g Chlorhexidin sowie 0,01 g Natriumfluorid durch nochmaliges Schütteln in der Flüssigkeit gelöst. Der erhaltene Lack wurde in einem verschlossenen Kunststoffgefäß bei Zimmertemperatur aufbewahrt.

## Beispiel 2

Die Wirkung des Lacks gemäß Beispiel 1 auf das Dentin gesunder und kariöser Rinderzähne wurde wie folgt untersucht:
Rinderzähne wurden zunächst 14 Tage lang bei 37 °C in einer künstlichen Karieslösung aufbewahrt. Die Lösung enthielt 6 Gew.% Carboxymethylcellulose und der pH-Wert betrug 4,5. Die Zähne waren während der gesamten Zeit vollständig mit Lösung bedeckt. Die Zähne wurden anschließend entnommen, mit Wasser gespült und getrocknet.

In zwei aufeinanderfolgenden Versuchen wurden jeweils 5 der mit Karieslösung behandelten Zähne sowie 5 unbehandelte, gesunde Rinderzähne mit dem gemäß Beispiel 1 hergestellten Lack überzogen. Die Wirkung des Lacks auf die Dentinhärte wurde ermittelt, indem man die Knoophärte (500 g Last) der mit Lack überzogenen Zähne im Vergleich zu den entsprechenden, nicht mit Lack behandelten Zähnen ermittelte.

Die Ergebnisse sind in Tabelle 1 wiedergegeben.

### Tabelle 1

Knoophärte Messung, Länge des Eindrucks in µm

| Versuch | Dentin ohne Karies | | Dentin mit Karies | |
|---|---|---|---|---|
| | ohne Lack | mit Lack | ohne Lack | mit Lack |
| 1 | 358 ± 25 | 350 ± 18 | 1233 ± 96 | 970 ± 71 |
| 2 | 356 ± 24 | 343 ± 29 | 1200 ± 75 | 1063 ± 61 |

Die Ergebnisse zeigen, daß die Härte des gesunden, nicht behandelten Dentins durch den erfindungsgemäßen Lack gemäß Beispiel 1 nicht oder nur unwesentlich beeinflußt wird. Demgegenüber wird die Härte des kariösen Dentins durch die Lackbehandlung deutlich gesteigert.

## Beispiel 3

Die antimikrobielle Wirksamkeit der erfindungsgemäßen Wirkstoffkombination wurde im Agardiffusionstest mit Streptococcus mutans nachgewiesen.

Es wurden die folgenden Testlösungen verwendet:

A) Lack gemäß Beispiel 1
B) Lack gemäß Beispiel 1 ohne Fluor-Ionen
C) Lack gemäß Beispiel 1 ohne Thymol
D) Lack gemäß Beispiel 1 ohne Chlorhexidin

0,05 ml jeder Lösung wurden auf mit Streptococcus mutans beimpfte Agarplatten auf einer Petrischale gebracht und die Durchmesser der Hemmhöfe nach 18 stündiger Inkubation der Platten bei 37 °C gemessen. Die Ergebnisse sind nachfolgend in Tabelle 2 wiedergegeben.

**Tabelle 2**

| Testlösung | Hemmhofdurchmesser, mm |
|---|---|
| A | 20 |
| B | 20 |
| C | 15 |
| D | 13 |

Die in Tabelle 2 wiedergegebenen Ergebnisse zeigen, daß der antibakterielle Effekt der Kombination von Thymol und Chlorhexidin gegenüber demjenigen der einzelnen Komponenten allein erheblich gesteigert ist. Demgegenüber beeinflußte die Anwesenheit von Fluor-Ionen den Hemmhofdurchmesser nicht.

## Patentansprüche

### Patentansprüche für folgende Vertragsstaaten : BE, CH, DE, ES, FR, GB, GR, IT, LI, LU, NL, SE

1. Verwendung einer Kombination von Thymol und/oder Carvacrol mit Chlorhexidin und/oder von physiologisch verträglichen Salzen derselben in einem dentalen Werkstoff als Träger zur Bekämpfung von Karies und Parodontose, wobei das Thymol und/oder Carvacrol oder deren Salze in einer Menge von 10 bis 0,05 Gew.% und das Chlorhexidin oder dessen Salze in einer Menge von 2 bis 0,01 Gew.%, jeweils bezogen auf den dentalen Werkstoff, vorliegen.

2. Verwendung nach Anspruch 1, **dadurch gekennzeichnet,** daß das Thymol und/oder Carvacrol oder deren Salze in einer Menge von 5 bis 0,25, vorzugsweise 2 bis 0,5 Gew.% und das Chlorhexidin oder dessen Salze in einer Menge von 1 bis 0,05, vorzugsweise 0,5 bis 0,1 Gew.% vorliegen.

3. Verwendung nach einem der Ansprüche 1 oder 2, **dadurch gekennzeichnet,** daß der dentale Werkstoff zusätzlich Fluor-Ionen enthält.

4. Verwendung nach einem der Ansprüche 1 bis 3, **dadurch gekennzeichnet,** daß der dentale Werkstoff ein Zahnlack, Dentalzement, Unterfüllungs-, Prothesen-, Kronen-, Brücken-, Füllungs-, Versiegelungs- oder Befestigungsmaterial oder ein Adhesiv ist.

5. Verwendung nach Anspruch 4, **dadurch gekennzeichnet**, daß der dentale Werkstoff ein Zahnlack auf Basis einer Lösung von Kolophonium in einem organischen Lösungsmittel ist.

6. Verwendung nach Anspruch 5, **dadurch gekennzeichnet,** daß das organische Lösungsmittel Ethanol, Propanol oder Amylacetat ist.

7. Verwendung nach den Ansprüchen 1 bis 6, **dadurch gekennzeichnet,** daß man die Kombination von Thymol und/oder Carvacrol mit Chlorhexidin und/oder von physiologisch verträglichen Salzen derselben in einem dentalen Werkstoff auf ein festes Substrat aufträgt, welches zum Einbringen in die Mundhöhle geeignet ist.

**8.** Verwendung nach Anspruch 7, **dadurch gekennzeichnet,** daß das feste Substrat ein Zahnstocher ist.

**Patentansprüche für folgenden Vertragsstaat : AT**

**1.** Verfahren zur Herstellung eines Mittels zur Bekämpfung von Karies und Parodontose, **dadurch gekennzeichnet,** daß man eine Kombination von Thymol und/oder Carvacrol mit Chlorhexidin und/oder von physiologisch verträglichen Salzen derselben in einen dentalen Werkstoff als Träger einbringt, wobei das Thymol und/oder Carvacrol oder deren Salze in einer Menge von 10 bis 0,05 Gew.% und das Chlorhexidin oder dessen Salze in einer Menge von 2 bis 0,01 Gew.%, jeweils bezogen auf den dentalen Werkstoff, vorliegen.

**2.** Verfahren nach Anspruch 1, **dadurch gekennzeichnet,** daß das Thymol und/oder Carvacrol oder deren Salze in einer Menge von 5 bis 0,25, vorzugsweise 2 bis 0,5 Gew.% und das Chlorhexidin oder dessen Salze in einer Menge von 1 bis 0,05, vorzugsweise 0,5 bis 0,1 Gew.% vorliegen.

**3.** Verfahren nach einem der Ansprüche 1 oder 2, **dadurch gekennzeichnet,** daß der dentale Werkstoff zusätzlich Fluor-Ionen enthält.

**4.** Verfahren nach einem der Ansprüche 1 bis 3, **dadurch gekennzeichnet,** daß der dentale Werkstoff ein Zahnlack, Dentalzement, Unterfüllungs-, Prothesen-, Kronen-, Brücken-, Füllungs-, Versiegelungs- oder Befestigungsmaterial oder ein Adhesiv ist.

**5.** Verfahren nach Anspruch 4, **dadurch gekennzeichnet,** daß der dentale Werkstoff ein Zahnlack auf Basis einer Lösung von Kolophonium in einem organischen Lösungsmittel ist.

**6.** Verfahren nach Anspruch 5, **dadurch gekennzeichnet,** daß das organische Lösungsmittel Ethanol, Propanol oder Amylacetat ist.

**7.** Verfahren nach den Ansprüchen 1 bis 6, **dadurch gekennzeichnet,** daß man die Kombination von Thymol und/oder Carvacrol mit Chlorhexidin und/oder von physiologisch verträglichen Salzen derselben in einem dentalen Werkstoff auf ein festes Substrat aufträgt, welches zum Einbringen in die Mundhöhle geeignet ist.

**8.** Verfahren nach Anspruch 7, **dadurch gekennzeichnet,** daß das feste Substrat ein Zahnstocher ist.

**Claims**

**Claims for the following Contracting States : BE, CH, DE, ES, FR, GB, GR, IT, LI, LU, NL, SE**

**1.** Use of a combination of thymol and/or carvacrol with chlorohexidine and/or of physiologically compatible salts thereof in a dental material as carrier for combatting caries and periodontosis in which the thymol and/or carvacrol or salts thereof are present in a quantity of 10 to 0.05 wt.-% and the chlorohexidine or its salts in a quantity of 2 to 0.01 wt.-%, relative in each case to the dental material.

**2.** Use according to claim 1, **characterized in that** the thymol and/or carvacrol or salts thereof are present in a quantity of 5 to 0.25, preferably 2 to 0.5 wt.-% and the chlorohexidine or its salts in a quantity of 1 to 0.05, preferably 0.5 to 0.1 wt.-%.

**3.** Use according to one of claims 1 or 2, **characterized in that** the dental material additionally contains fluorine ions.

**4.** Use according to one of claims 1 to 3, **characterized in that** the dental material is a tooth varnish, dental cement, underfilling, prosthesis, crown, bridge, filling, sealing or securing material or an adhesive.

**5.** Use according to claim 4, **characterized in that** the dental material is a tooth varnish based on a solution of colophony in an organic solvent.

**6.** Use according to claim 5, **characterized in that** the organic solvent is ethanol, propanol or amyl

acetate.

**7.** Use according to claims 1 to 6, **characterized in that** the combination of thymol and/or carvacrol with chlorohexidine and/or of physiologically compatible salts thereof is deposited in a dental material onto a solid substrate which is suitable for introduction into the oral cavity.

**8.** Use according to claim 7, **characterized in that** the solid substrate is a toothpick.

**Claims for the following Contracting State : AT**

**1.** A process for the preparation of a composition for combatting caries and periodontosis, comprising incorporating a combination of thymol and/or carvacrol with chlorohexidine and/or of physiologically compatible salts thereof in a dental material as carrier in which the thymol and/or carvacrol or salts thereof are present in a quantity of 10 to 0.05 wt.% and the chlorohexidine or its salts in a quantity of 2 to 0.01 wt.%, relative in each case to the dental material.

**2.** A process according to claim 1, characterized in that the thymol and/or carvacrol or salts thereof are present in a quantity of 5 to 0.25, preferably 2 to 0.5 wt.% and the chlorohexidine or its salts in a quantity of 1 to 0.05, preferably 0.5 to 0.1 wt.%.

**3.** A process according to one of claims 1 or 2, characterized in that the dental material additionally contains fluorine ions.

**4.** A process according to one of claims 1 to 3, characterized in that the dental material is a tooth varnish, dental cement, underfilling, prosthesis, crown, bridge, filling, sealing or securing material or an adhesive.

**5.** A process according to claim 4, characterized in that the dental material is a tooth varnish based on a solution of colophony in an organic solvent.

**6.** A process according to claim 5, characterized in that the organic solvent is ethanol, propanol or amyl acetate.

**7.** A process according to claims 1 to 6, characterized in that the combination of thymol and/or carvacrol with chlorohexidine and/or of physiologically compatible salts thereof is deposited in a dental material onto a solid substrate which is suitable for introduction into the oral cavity.

**8.** A process according to claim 7, characterized in that the solid substrate is a toothpick.

**Revendications**
**Revendications pour les Etats contractants suivants : BE, CH, DE, ES, FR, GB, GR, IT, LI, LU, NL, SE**

**1.** Utilisation d'une combinaison de thymol et/ou de carvacrol avec de la chlorhexidine et/ou leurs sels physiologiquement compatibles, dans un matériau dentaire support, pour lutter contre les caries et la parodontose, combinaison dans laquelle le thymol et/ou le carvacrol ou leurs sels est (sont) présent(s) à raison de 10 à 0,05 % en poids et la chlorhexidine ou ses sels est (sont) présent(s) à raison de 2 à 0,01 % en poids, chaque fois par rapport au matériau dentaire.

**2.** Utilisation selon la revendication 1, caractérisée en ce que le thymol et/ou le carvacrol ou leurs sels est (sont) présent(s) à raison de 5 à 0,25, de préférence de 2 à 0,5, % en poids, et la chlorhexidine ou ses sels est (sont) présent(s) à raison de 1 à 0,05, de préférence de 0,5 à 0,1, % en poids.

**3.** Utilisation selon une des revendications 1 ou 2, caractérisée en ce que le matériau dentaire contient en outre des ions fluor.

**4.** Utilisation selon une des revendications 1 à 3, caractérisée en ce que le matériau dentaire est une laque dentaire, un ciment dentaire, un matériau d'obturation de base, un matériau pour prothèses, pour couronnes, pour ponts (bridges), ou un matériau d'obturation, de scellement ou de consolidation, ou

bien un adhésif.

5. Utilisation selon la revendication 4, caractérisée en ce que le matériau dentaire est une laque dentaire à base d'une solution de colophane dans un solvant organique.

6. Utilisation selon la revendication 5, caractérisée en ce que le solvant organique est l'éthanol, le propanol ou l'acétate d'amyle.

7. Utilisation selon les revendications 1 à 6, caractérisée en ce qu'on dépose la combinaison, dans un matériau dentaire, de thymol et/ou de carvacrol avec de la chlorhexidine et/ou leurs sels physiologiquement compatibles sur un substrat solide, approprié pour être introduit dans la cavité buccale.

8. Utilisation selon la revendication 7, caractérisée en ce que le substrat solide est un cure-dents.

**Revendications pour l'Etat contractant suivant : AT**

1. Procédé pour la fabrication d'un composer pour lutter contre les caries et la parodontose caractérisé en ce qu'on incorpore une combination de thymol et/ou de carvacrol avec de la chlorhexidine et/ou leurs sels physiologiquement compatibles, dans un matériau dentaire support, combinaison dans laquelle le thymol et/ou le carvacrol ou leurs sels est (sont) présent(s) à raison de 10 à 0,05 % en poids et la chlorhexidine ou ses sels est (sont) présent(s) à raison de 2 à 0,01 % en poids, chaque fois par rapport au matériau dentaire.

2. Procédé selon la revendication 1, caractérisé en ce que le thymol et/ou le carvacrol ou leurs sels est (sont) présent(s) à raison de 5 à 0,25, de préférence de 2 à 0,5, % en poids, et la chlorhexidine ou ses sels est (sont) présent à raison de 1 à 0,05, de préférence de 0,5 à 0,1, % en poids.

3. Procédé selon une des revendications 1 ou 2, caractérisé en ce que le matériau dentaire contient en outre des ions fluor.

4. Procédé selon une des revendications 1 à 3, caractérisé en ce que le matériau dentaire est une laque dentaire, un ciment dentaire, un matériau d'obturation de base, un matériau pour prothèses, pour couronnes, pour ponts (bridges), ou un matériau d'obturation, de scellement ou de consolidation, ou bien un adhésif.

5. Procédé selon la revendication 4, caractérisé en ce que le matériau dentaire est une laque dentaire à base d'une solution de colophane dans un solvant organique.

6. Procédé selon la revendication 5, caratérisé en ce que le solvant organique est l'éthanol, le propanol ou l'acétate d'amyle.

7. Procédé selon les revendications 1 à 6, caractérisé en ce qu'on dépose la combinaison, dans un matériau dentaire, de thymol et/ou de carvacrol avec de la chlorhexidine et/ou leurs sels physiologiquement compatibles sur un substrat solide, approprié pour être introduit dans la cavité buccale.

8. Procédé selon la revendication 7, caractérisé en ce que le substrat solide est un cure-dents.